# EUROPÄISCHE PATENTSCHRIFT

(11) **EP 2 847 317 B1**
(45) Veröffentlichungstag und Bekanntmachung des Hinweises auf die Patenterteilung: **21.04.2021**
(21) Anmeldenummer: 13723080.1
(22) Anmeldetag: 13.05.2013
(51) Int. Cl.: C12M 1/00, A61M 1/36, B01D 21/26, B04B 5/04

(54) **ELUTRIATIONSKAMMER FÜR EIN ELUTRIATORSYSTEM**
ELUTRIATION CHAMBER FOR AN ELUTRIATOR SYSTEM
CHAMBRE D'ÉLUTRIATION POUR SYSTÈME ÉLUTRIATEUR

(30) Priorität: 11.05.2012 AT 501732012
(43) Veröffentlichungstag der Anmeldung: 18.03.2015
(73) Patentinhaber: Biomedizinische Forschung & Bio-Produkte AG, 1090 Wien (AT)
(72) Erfinder: EIBL, Johann, A-1180 Wien (AT); GRAUS, Johann, A-1030 Wien (AT); MADER, Christoph, A-2100 Korneuburg (AT)
(74) Vertreter: Schwarz & Partner Patentanwälte OG
(86) Internationale Anmeldenummer: PCT/EP2013/059761
(87) Internationale Veröffentlichungsnummer: WO 2013/167747

(56) Entgegenhaltungen:
- EP-A2- 2 169 380
- WO-A1-99/23471
- DE-A1- 2 701 976
- US-A- 4 322 298
- US-A- 4 939 087
- US-A1- 2006 147 895
- US-A1- 2008 318 756

## Beschreibung

Die Erfindung betrifft eine Elutriationskammer für ein Elutriatorsystem zum Waschen und/oder Isolieren von Zellen, insbesondere Thrombozyten.

### Einleitung

Die Gewinnung menschlicher Blutzellen von geeigneten Blutspendern durch Einsatz von Zentrifugen war bisher darauf ausgerichtet, in einem möglichst kontinuierlichen Prozess Blut nach Austritt aus dem Spender in einem Schlauchsystem und einer entsprechenden Zentrifuge in Blutbestandteile aufzutrennen, den gewünschten Bestandteil zu isolieren und alle anderen Bestandteile unmittelbar wieder in den Spender rückzuführen. Dieser Prozess wird als Apherese bezeichnet. Durch die Plasmapherese wurde es möglich, unter größter Schonung des Spenders große Mengen von Blutplasma für die Herstellung von Medikamenten zur Behandlung schwerer Erkrankungen zu gewinnen. Analog zur Plasmapherese konnten auch benötigte Mengen von Thrombozyten-reichem Plasma durch Thrombapherese gewonnen werden.

Zur Gewinnung von Plasma und Thrombozyten war die Konstruktion spezieller Durchlaufzentrifugen erforderlich, die auch zur Gewinnung und Reinigung von anderen menschlichen Zellen, insbesondere Knochenmarkzellen, geeignet waren.

### Aufgabenstellung

Thrombozyten sind im Vergleich zu den meisten anderen Blutzellen kleine Zellen, die keinen Zellkern besitzen. Durchschnittlich sind im zirkulierenden Blut 200000 bis 300000 Thrombozyten pro µl vorhanden. Bei Thrombozytopenien kann die Anzahl der Thrombozyten zunächst auf 40000 Zellen pro µl zurückgehen, ohne dass es zu einer auffälligen Symptomatik kommt. Wenn die Thrombozytenzahl weiter absinkt, insbesondere in einen Bereich von 1000 bis 10000 Thrombozyten pro µl, können mehr oder weniger starke, schwer stillbare Blutungen auftreten. Solche Blutungen können zum Verblutungstod führen.

Thrombozytopenien können durch verschiedene Grunderkrankungen entstehen oder durch Medikamente hervorgerufen werden, insbesondere durch Zytostatika. Die Behandlung von Thrombozytopenien konnte zunächst durch Transfusion von frischen Blutkonserven erfolgreich vorgenommen werden. Der Nachteil dieser Behandlung bestand darin, dass man Patienten nur beschränkt Blut transfundieren kann, und so längerfristig nicht die erforderliche Anzahl von Thrombozyten im zirkulierenden Blut erreicht.

Nachdem es gelungen war, aus Blutkonserven unmittelbar nach der Blutabnahme plättchenreiches Plasma zu gewinnen, bestand die Möglichkeit der häufigeren Anwendung und eine ausreichende Behandlung auch schwerer Thrombozytopenien. Die Situation wurde weiter verbessert, nachdem es mittels Thrombapherese gelang, Thrombozyten-reiches Plasma statt Vollblut zu gewinnen. Dadurch konnte die Frequenz der Spenden bei den einzelnen Blutspendern erhöht werden.

Patienten mit schweren Thrombozytopenien, die häufig Infusionen von Thrombozyten-reichem Plasma erhalten, können sehr oft schwere Nebenwirkungen erleiden. Ein Teil dieser Nebenwirkungen kann auf die Infusion von Thrombozyten zurückgeführt werden, ein anderer Teil auf das im Thrombozyten-reichen Plasma noch enthaltene Blutplasma. Um dieses Plasma zu entfernen, wurde versucht, durch Zentrifugation die Plättchen zu pelletieren, den plasmahältigen Überstand zu entfernen, und das Plättchenpellet wieder in geeigneten Pufferlösungen zu resuspendieren. Diese Vorgangsweise hat aber den Nachteil, dass pelletierte Plättchen schwierig gut zu resuspendieren sind, sodass Plättchenaggregate entstehen, die nicht mehr in einzelne Plättchen aufzulösen sind. Durch die Aggregation der Plättchen kann es auch zu einer mehr oder weniger starken Aktivierung der Plättchen kommen und damit auch zu einer Erhöhung der Gefahr der Thrombenbildung. Aus diesen Umständen heraus erscheint es wünschenswert, Plasma und mikrobielle Verunreinigungen, die im Plasma vorkommen können, durch einen verbesserten Waschprozess von den Plättchen abzutrennen. Ein solcher Abtrennungsprozess soll ferner möglichst jegliche Aktivierung der Plättchen vermeiden, voll aseptisch durchführbar sein und auch noch vorhandene andere Zellen, insbesondere weiße Blutkörperchen, entfernen.

### Stand der Technik

Für wissenschaftliche Zwecke und für die Herstellung von Zellsuspensionen für medizinische Anwendungen sind bereits eine Reihe von Geräten und Apparaturen kommerziell erhältlich.

Damit können sowohl autologe wie homologe Zellpräparationen aus Blut und Geweben gewonnen werden. Solche Geräte und Apparaturen dienen der Gewinnung bzw. Reinigung von Zellen in Durchlaufzentrifugen.

Bei der Gewinnung von Plasma durch Plasmapherese kann das gewonnene Plasma noch mit verschiedenen Blutzellen verunreinigt sein. Solche Verunreinigungen stellen aber für die Verwendung zur Herstellung von Arzneimitteln kein Hindernis dar. Die durch Thrombapherese gewonnenen plättchenreichen Plasmapräparationen weisen aber mehrere Nachteile auf, insbesondere die Verunreinigung mit anderen Blutzellen. Auch das in dem plättchenreichen Plasma enthaltene Plasma kann bei der Anwendung am Menschen zu Nachteilen führen.

Die Entfernung weißer Blutzellen, die sich im plättchenreichen Plasma befinden, durch Filtration ist keine befriedigende Lösung. Die Leukozyten werden zwar am Filter adsorbiert und dadurch entfernt, aber nach ihrer Adsorption zerstört. Dadurch tritt der Zellinhalt der Leukozyten wieder in das Filtrat und somit in das Thrombozytenkonzentrat.

Die Entfernung von Plasma aus dem plättchenreichen Plasma durch Zentrifugation soll nach Möglichkeit vermieden werden. Durch die Sedimentation der Thrombozyten kann zwar das überstehende Plasma weitestgehend entfernt werden, die sedimentierten Thrombozyten bilden aber ein Pellet, das schwer vollständig resuspendierbar ist. Darüberhinaus ist der überwiegende Teil der resuspendierten Thrombozyten aggregiert und zum Teil auch aktiviert. Bei der intravenösen Anwendung solcher Thrombozytensuspensionen besteht dadurch eine erhöhte Thrombosegefahr für den Empfänger.

Zur Trennung und Isolierung dieser Zellen ist die sogenannte Elutriation bekannt, die auf dem Prinzip der Gegenstromzentrifugation beruht (z.B. US 5,674,173, EP 2 169 380 A2 und EP 0 824 380 B1).

Dabei sind zwei gegeneinanderwirkende Kräfte von Bedeutung: Zum einen die nach außen gerichtete Zentrifugalkraft, die durch die Rotationsgeschwindigkeit des Elutriatorrotors bestimmt wird und zum anderen die Zentripetalkraft, die durch die Strömungsgeschwindigkeit des Mediums in Richtung der Rotationsachse mittels einer Pumpe bestimmt wird. Befinden sich beide Kräfte im Gleichgewicht, können Partikel bestimmter Größe und Dichte in der Elutriationskammer konzentriert werden, während die übrigen ausgewaschen werden.

### Aufgabenlösung

Zur Abtrennung von Thrombozyten aus plättchenreichem Plasma wird in einer Durchlaufzentrifuge der Zufluss zur Elutriationskammer mit einem entsprechend dimensionierten Schlauch ausgestattet. Die Strömungsgeschwindigkeit in diesem Schlauch bei einer gegebenen Umdrehungszahl der Zentrifuge wird während des Gesamtvorganges so hoch gehalten, dass es zu keinem thrombozytenreichen Sediment im Zuführungsschlauch kommt. Dadurch wird ein unnötiger Verlust an Thrombozyten vermieden und können Leukozyten entfernt werden.

Die Einflussöffnung in die Elutriationskammer wird bevorzugt so klein gehalten, dass die Einflussgeschwindigkeit des thrombozytenreichen Plasmas etwa 5 m/s. beträgt. Dadurch ist es möglich, den Corioliseffekt zu unterbinden, der die Thrombozyten an die Trichterwand der Elutriationskammer führt und entlang der Trichterwand unmittelbar in den Abfluss der Elutriationskammer, wodurch der Hauptteil der Thrombozyten verloren geht.

Das bei hoher Strömungsgeschwindigkeit in die Elutriationskammer eingebrachte thrombozytenreiche Plasma führt sehr rasch zur Ausbildung einer thrombozytenreichen Mittelschicht in der Elutriationskammer. Nach Einbringung des gesamten thrombozytenreichen Plasmas können die Thrombozyten sehr gut durch gleichartige Zufuhr von geeigneten Pufferlösungen ohne wesentlichen Verlust gewaschen werden.

Durch Anbringung einer zweiten Einflussöffnung im oberen Trichter der Elutriationskammer kann die Thrombozytenschicht gewonnen werden, ohne dass bei der Eintrittsöffnung der Elutriationskammer Flüssigkeit aus dem zuführenden Schlauch übertritt.

Der einzelne Elutriationsvorgang kann dabei auch aseptisch durchgeführt werden.

Die erfindungsgemäße Elutrationskammer für ein Elutriatorsystem zum Waschen und/oder Isolieren von Zellen, insbesondere Thrombozyten, welche Elutriationskammer eine Zuleitung (1) für ein wässeriges Medium, welches die zu waschenden und/oder zu isolierenden Zellen suspendiert enthält, und eine Ableitung (2) für die gewaschenen und/oder isolierten Zellen aufweist, wobei die Kammer (5) bevorzugt rotationssymmetrisch zur Achse (a) ist, ist dadurch gekennzeichnet, dass das Verhältnis der Fläche des Schnittes durch das Lumen der Kammer (5) normal zur Achse (a) an der weitesten Stelle (5a-5b) zur Fläche des Schnittes (1a) durch die Zuleitung (1) im Bereich von 1000 bis 250000 liegt.

Eine weitere Elutriationskammer weist eine weitere Zuleitung (3) für ein gasförmiges oder flüssiges Medium auf.

Eine bevorzugte Ausführungsform einer erfindungsgemäßen Elutriationskammer ist dadurch gekennzeichnet, daß das Verhältnis der Fläche des Schnittes durch das Lumen der Kammer (5) normal zur Achse (a) an der weitesten Stelle (5a-5b) zur Fläche des Schnittes (1a) durch die Zuleitung (1) im Bereich von 1000 bis 250000 liegt und daß sie eine weitere Zuleitung (3) für ein gasförmiges oder flüssiges Medium aufweist.

Eine weitere erfindungsgemäße Elutriationskammer für ein Elutriatorsystem ist schließlich dadurch gekennzeichnet, dass zwischen der Zuleitung (1) und der Kammer (5) eine Verengung (4) vorgesehen ist, die vorzugsweise konisch ausgeführt ist, derart, dass das Verhältnis zwischen der Fläche des Schnittes durch das Lumen der Kammer (5) normal zur Achse (a) an der weitesten Stelle (5a-5b) zur Fläche des Schnittes durch die Verengung (4) im Bereich von 1000 bis 250000 liegt. Diese Verengung wirkt als Düse und beschleunigt den in die Kammer eintretenden Flüssigkeitsstrahl.

Weitere bevorzugte Ausführungsformen der erfindungsgemäßen Elutriationskammer sind in den beigefügten Patentansprüchen angegeben.

Die Erfindung betrifft schließlich noch ein Verfahren zur Entfernung unerwünschter Kontaminanten, wie Mikropartikel, mikrobielle Pathogene, Plasmaproteine, photodynamische und andere virusinaktivierende Mittel von Thrombozyten, wobei eine erfindungsgemäße Elutriationskammer verwendet wird, mit der Maßgabe, daß bei diesem Verfahren die Thrombozyten nicht pelletiert werden.

Bevorzugte Ausführungsformen werden an Hand der beigefügten Zeichnung näher beschrieben.

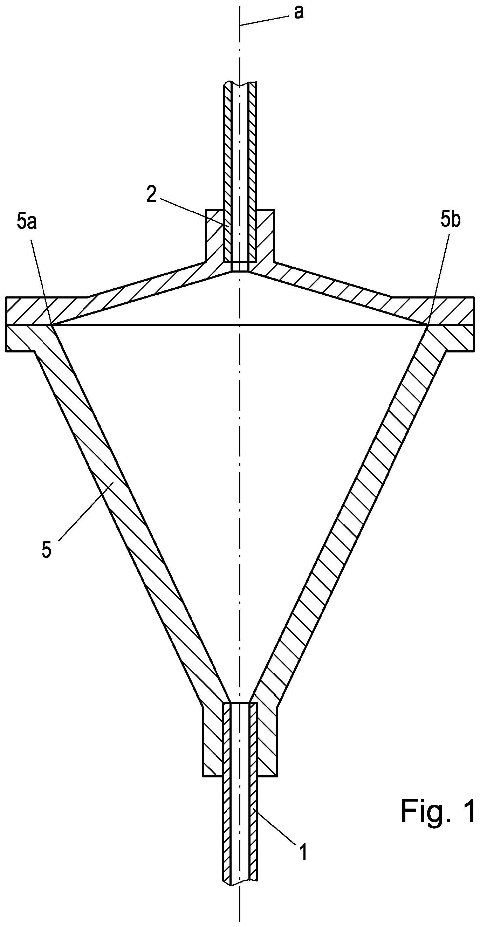

Die Figur 1 zeigt eine in einem kommerziell erhältlichen Elutriatorsystem enthaltene Eluatrionskammer.

Diese ist mit ihrer Symmetrieachse entlang des Radius auf dem Zentrifugenrotor verankert, sodass sich der Fluidzulauf (1) nahe dem Rotorumfang und entsprechend der Fluidablauf (2) in Achsennähe befinden. Während des Betriebs der Zentrifuge können nun die entsprechenden Fluidströme über die Rotorachse zu bzw. abgeführt werden und generieren in der Kammer den der Zentrifugalbeschleunigung entgegengerichteten Strom. Auf Grund des sich in zentripedaler Richtung erweiternden Kammerquerschnitts und der damit verbundenen, sich kontinuierlich verringernden Strömungsgeschwindigkeit kann sich innerhalb gewisser Grenzen ein Gleichgewicht zwischen der auf ein Partikel wirkenden Zentrifugal- und Widerstandskraft einstellen. Dadurch werden Partikel eines entsprechenden Bereichs an Sedimentationsgeschwindigkeiten im System in Schwebe gehalten, während die restlichen Anteile des Fluids kontinuierlich ausgewaschen werden.

Die konstruktive Anbindung des Elutriationskammerzu- und -ablaufs über die Rotationsachse bedingt einen Zulaufkanal, der vom Rotorzentrum kommend unmittelbar vor der Trennkammer an der Rotorperipherie in eine radial zur Rotorachse hin verlaufende Richtung umgelenkt werden muß. Da dabei die Kanalwandung an einer Stelle normal zu der aus Sedimentations- und Strömungsgeschwindigkeit resultierenden Teilchenbewegung verlaufen muß, neigt dieser Bereich insbesonders bei zu Verklumpung neigenden Partikeln, wie etwa Thrombozyten, zu einer massiven Pelletbildung, was sich für die vorgesehene Anwendung nachteilig auswirkt.

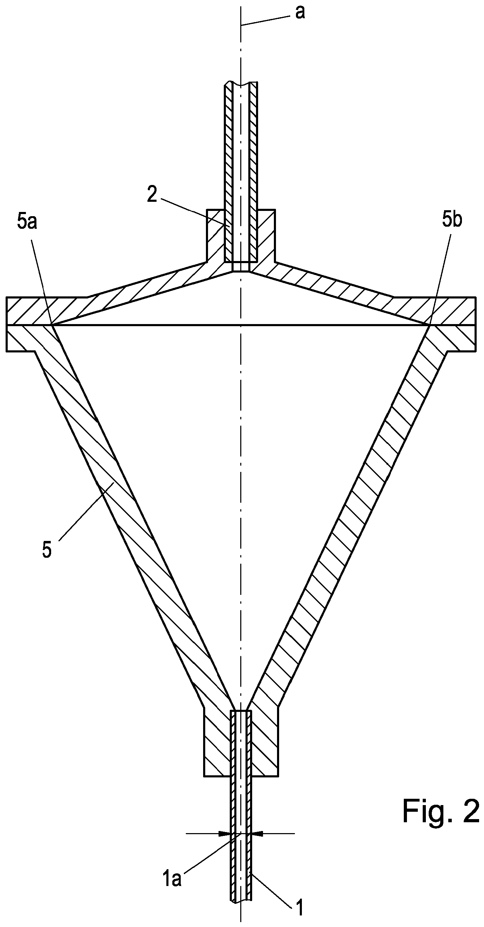

Die Figur 2 zeigt eine erfindungsgemäße Elutriationskammer, bei welcher die Zuleitung (1) einen derart engen Querschnitt aufweist, dass bei der angewandten Flussrate eine Durchtrittsgeschwindigkeit von mehr als 2 cm/s vorzugsweise >10 cm/s (7 bis 16 cm/s) erreicht wird. Durch die Erhöhung der Fließgeschwindigkeit durch einen reduzierten Leitungsquerschnitt wird der Pelletbildung in dem Bereich der Umlenkung des zugeführten Fluidstromes in die zentripedale Richtung unmittelbar vor der Trennkammer entgegengewirkt. Entscheidend ist dabei, dass das Verhältnis der Fläche des Schnittes durch das Lumen der Kammer (5) normal zur Achse (a) an der weitesten Stelle (5a-5b) zur Fläche des Schnittes (1a) durch die Zuleitung (1) im Bereich von 1000 bis 250000 liegt.

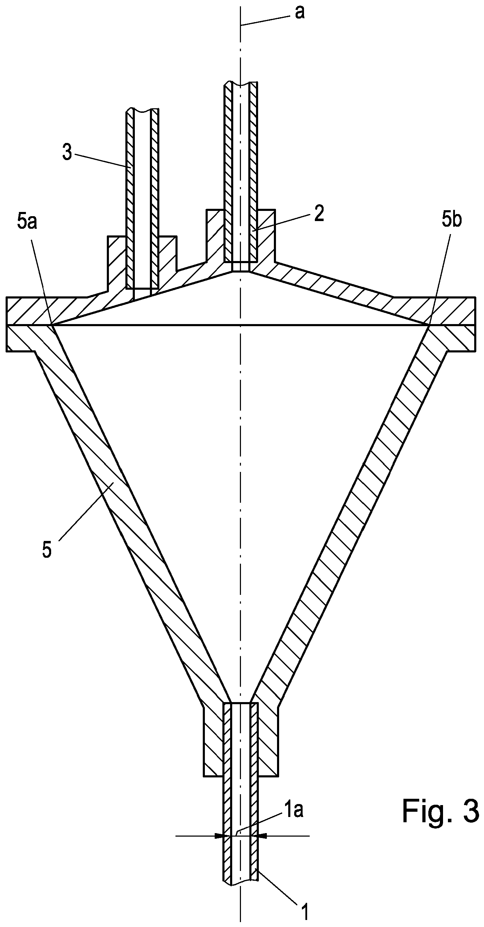

Die Figur 3 zeigt eine weitere erfindungsgemäße Elutriationskammer, welche eine weitere von der normalen, der Beladung und dem Gegenstrom dienenden Zuleitung (1) unabhängige direkte Zuführung (3) aufweist. Diese dient zu Prozessende als sauberer Zulauf zur Gewinnung des Kammerinhalts, ohne dass im normalen Zulauf sedimentiertes Material in die Kammer verschleppt und mit deren Inhalt vermischt wird.

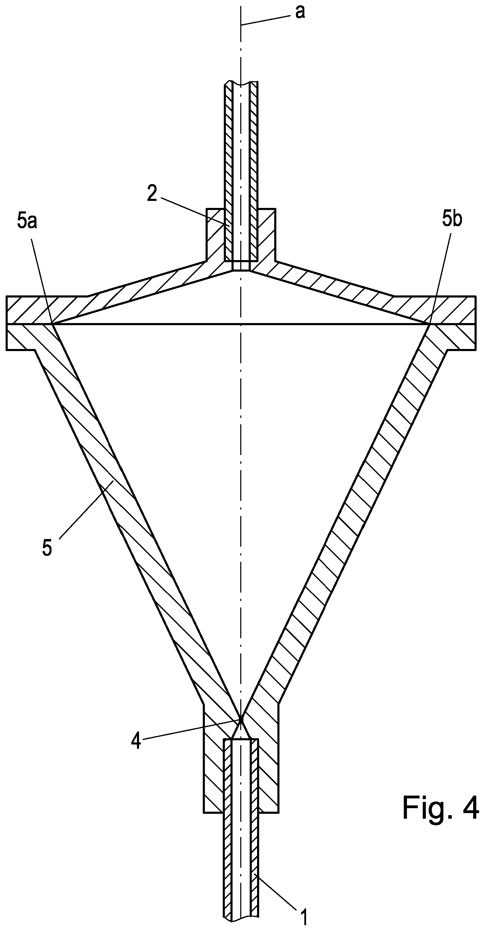

Die Figur 4 zeigt eine zusätzliche erfindungsgemäße Elutriatrionskammer, bei welcher in der Zuleitung (1) eine starke Verengung des Einlaufquerschnittes (4) vorgesehen ist. Dies erlaubt durch einfache Unterbrechung des Zuflusses nach erfolgter Elutriation eine nahezu vollständige und effektive Separation des Kammerlumens von der normalen Zulaufleitung und verhindert so einer Verschleppung von dort sedimentiertem Material durch Diffusion oder kleine Druckschwankungen. Während der Elutriation sorgt die Verengung des Zulaufs für eine Reduktion des Ausmaßes der durch die Corioliskraft bedingten kriechenden Rückströmung von der Kammer in die Zulaufleitung entlang der von der Drehrichtung abgewandten Wandung. Weiters kommt es durch den mit hoher Geschwindigkeit eintretenden Strahl und die damit im Einlaufbereich verbundene Verwirbelung zu einer Dispersion des einströmenden Fluids über den Kammerquerschnitt, was einerseits zu einer wirksamen Waschung beiträgt und andererseits der Bildung einer Kanalströmung entlang der in Drehrichtung befindlichen Kammerwand in Folge der Corioliskraft entgegenwirkt.

Eine bevorzugte Ausführungsform der erfindungsgemäßen Elutriationskammer ist im nachfolgenden Beispiel beschrieben und besteht in einer Kombination aller drei in den Figuren 2, 3 und 4 dargestellten Varianten.

### Beispiel

Die aus transparentem Kunststoff gefertigte Elutriationskammer ist im Wesentlichen aus zwei an ihrer Basis verbundenen, geraden Hohlkegeln aufgebaut, sodass sich eine doppelkegelförmige Kammer ergibt. Diese weist ihre weiteste Stelle (5a-5b) mit einem Innendurchmesser von 50 mm am gemeinsamen Kreisquerschnitt auf, von welchem aus sich die beiden Hohlkegel 51 mm bzw. 8 mm zu ihren Spitzen in jeweils entgegengesetzter Richtung erstrecken.

Dieser Grundkörper ist mit seiner Symmetrieachse (a) im Zentrifugenrotor entlang des Radius derart ausgerichtet, dass die Spitze des niedrigen Kegels in Richtung Rotorachse und dementsprechend die des hohen Kegels zum Rotorumfang weist. Für den Zulauf (1) im Normalbetrieb ist an der peripher zur Rotorachse gelegenen Spitze des hohen Kegels eine Verengung (4) auf 0,3mm Durchmesser vorhanden, die sich nach außen hin konisch auf den Innendurchmesser des angeschlossenen Zulaufschlauches von 1,2 mm erweitert. Das Verhältnis der Fläche des Schnittes durch das Lumen der Kammer (5) normal zur Achse (a) an der weitesten Stelle (5a-5b) normal zur Achse (a) zur Fläche des Schnittes (1a) durch die Zuleitung (1) beträgt somit 1736 (1962 mm²/1,13 mm²). Das Verhältnis der Querschnittsflächen erhöht sich auf 27778 (1962 mm²/ 0,071 mm²), wenn eine Verengung auf 0,3 mm im Durchschnitt vorhanden ist.

An der dazu gegenüberliegenden, nahe der Rotorachse gelegenen Kegelspitze befindet sich die Auslauföffnung von 2,9 mm Durchmesser, die in den Ablaufschlauch gleichen Innendurchmessers mündet. Zur vom normalen Zulauf unabhängigen Entleerung des Kammerinhaltes enthält die Mantelfläche des flachen Hohlkegels 14 mm azentrisch zur Symmetrieachse zusätzlich eine weitere Einlauföffnung (3) von 2,9 mm Durchmesser, die mit dem entsprechenden Zulaufschlauch verbunden ist.

Dieser Zentrifugeneinsatz ist auf dem Rotor eines handelsüblichen Elutriationssystems installiert, das sowohl Schlauchquetschpumpen zur Gewährleistung der benötigten Flüssigkeitsströme als auch eine Verbindung zu den entsprechenden Zu- und Ablaufleitungen der rotierenden Kammer bereitstellt.

Die beiden Zulaufleitungen sind außerhalb des Rotors über einen Dreiweghahn zusammengeführt und in weiterer Folge über eine Tropfkammer und eine Pumpe mit einem Mehrweghahn zur Versorgung mit der jeweiligen Flüssigkeit verbunden. Die den Rotor verlassende Ablaufleitung führt zu zwei wechselseitig schaltbaren Behältnissen, einerseits zur Sammlung der prozessierten Fraktion und andererseits zur Aufnahme aller übrigen Flüssigkeiten.

Zu Prozessbeginn wird die Kammer und das gesamte Leitungssystem mit einer geeigneten Lösung gefüllt, die Zentrifuge auf eine Umdrehungszahl von 2600 min⁻¹ gebracht, und ein kontinuierlicher Strom von 5ml/min über den für den Normalbetrieb an der Rotorperipherie befindlichen Kammerzulauf etabliert, wobei die zweite zur Entleerung bestimmte Zulaufleitung beim Dreiweghahn verschlossen ist.

Nach Einstellung eines stationären Flusses durch das System wird die Kammer durch kontinuierliche Zufuhr des gesamten, in einer entsprechenden Pufferlösung verdünnten, thrombozytenreichen Plasmas beladen. Im Anschluss daran wird der Fluss mit einer geeigneten Waschlösung weiter aufrecht erhalten. Ist das erforderliche Ausmaß der Waschung erreicht, wird gleichzeitig der Fluss sowie die Zentrifuge gestoppt und über den Dreiweghahn eine Verbindung nur zweier Kammerzuläufe miteinander hergestellt. Nach Entnahme der Elutriationskammer aus der Rotorverankerung und ihrer Ausrichtung mit der Ablauföffnung nach unten, sowie Umschaltung der Ablaufleitung wird nun der Kammerinhalt ausschließlich über die zweite, zusätzliche Zulaufleitung mit Luft in das Sammelbehältnis für die prozessierte Fraktion gedrückt.

Die erfindungsgemäße Elutriationskammer gestattet die Gewinnung von Zellen und Zellbestandteilen, wobei der zuführende Schlauch so dimensioniert ist, dass kein thrombozytenreiches Pellet während des Elutriationsvorganges gebildet werden kann.

Die erfindungsgemäße Elutriationskammer ermöglicht ferner hohe Einströmungsgeschwindigkeiten der zu reinigenden Zellsuspension oder Suspension von Zellfragmenten zwischen 1 - 10 m/sec., vorzugsweise 5 m /sec.

Sind zwei Abflussöffnungen in dem oberen Trichterteil der Elutriationskammer vorgesehen, so gestattet diese Ausführungsform die Gewinnung elutriierter Zellen oder Zellbestandteile ohne Kontamination mit Partikeln, die sich noch im Zuführungsschlauch zur Elutriationskammer befinden.

Darüberhinaus gestattet die Verengung des Flüssigkeitseintritts in die Elutriationskammer eine hohe Strömungsgeschwindigkeit der Zellsuspensionen oder der Suspensionen von Zellbestandteilen zu erreichen und die Auswirkungen des Corioliseffektes zu minimieren.

## Patentansprüche

1. Elutriationskammer für ein Elutriatorsystem zum Waschen und/oder Isolieren von Zellen, insbesondere Thrombozyten, welche Elutriationskammer eine Zuleitung (1) für ein wässeriges Medium, welches die zu waschenden und/oder zu isolierenden Zellen suspendiert enthält, und eine Ableitung (2) für die gewaschenen und/oder isolierten Zellen aufweist, wobei die Kammer (5) bevorzugt rotationssymmetrisch zur Achse (a) ist,
**dadurch gekennzeichnet,**
**dass** das Verhältnis der Fläche des Schnittes durch das Lumen der Kammer (5) normal zur Achse (a) an der weitesten Stelle (5a-5b) zur Fläche des Schnittes (1a) durch die Zuleitung (1) im Bereich von 1000 bis 250000 liegt.

2. Elutriationskammer für ein Elutriatorsystem nach Anspruch 1,
**dadurch gekennzeichnet,**
**dass** sie eine weitere Zuleitung (3) für ein gasförmiges oder flüssiges Medium aufweist.

3. Elutriationskammer für ein Elutriatorsystem zum Waschen und/oder Isolieren von Zellen, insbesondere Thrombozyten, welche Elutriationskammer eine im Querschnitt kreisförmige Zuleitung (1) für ein wässeriges Medium, welches die zu waschenden und/oder zu isolierenden Zellen suspendiert enthält, und eine Ableitung (2) für die gewaschenen und/oder isolierten Zellen aufweist,
**dadurch gekennzeichnet,**
**dass** in der Zuleitung (1) beim Kammereintritt eine Verengung (4), die vorzugsweise konisch ist, vorgesehen ist, derart, dass das Verhältnis zwischen der Fläche des Schnittes durch das Lumen der Kammer (5) normal zur Achse (a) an der weitesten Stelle (5a-5b) zur Fläche des Schnittes durch die Verengung (4) im Bereich von 1000 bis 250000 liegt.

4. Elutriationskammer nach Anspruch 3 in Kombination mit Anspruch 1.

5. Elutriationskammer nach Anspruch 3 in Kombination mit Anspruch 2.

6. Verfahren zur Entfernung unerwünschter Kontaminanten, wie Mikropartikel, mikrobielle Pathogene, Plasmaproteine, photodynamische und andere virusinaktivierende Mittel von Thrombozyten, wobei eine Elutriationskammer nach einem der Ansprüche 1 bis 5 verwendet wird, mit der Maßgabe, daß die Thrombozyten nicht pelletiert werden.

## Claims

1. An elutriation chamber for an elutriator system for washing and/or isolating cells, in particular thrombocytes, which elutriation chamber comprises a feed line (1) for an aqueous medium containing the cells to be washed and/or to be isolated in suspended form, and a discharge line (2) for the washed and/or isolated cells, the chamber (5) preferably being rotationally symmetrical to the axis (a),
**characterized in that**
the ratio of the area of the section through the lumen of the chamber (5) perpendicular to the axis (a) at the widest point (5a-5b) to the area of the section (1a) through the feed line (1) is in the range of 1,000 to 250,000.

2. An elutriation chamber for an elutriator system according to claim 1, **characterized in that** it comprises a further feed line (3) for a gaseous or liquid medium.

3. An elutriation chamber for an elutriator system for washing and/or isolating cells, in particular thrombocytes, which elutriation chamber comprises a feed line (1) with a circular cross-section for an aqueous medium containing the cells to be washed and/or to be isolated in suspended form, and a discharge line (2) for the washed and/or isolated cells,
**characterized in that**,
in the feed line (1) at the chamber entrance, a narrowing (4), which preferably is conical, is provided in such a way that the ratio between the area of the section through the lumen of the chamber (5) perpendicular to the axis (a) at the widest point (5a-5b) to the area of the section through the narrowing (4) is in the range of 1,000 to 250,000.

4. An elutriation chamber according to claim 3 in combination with claim 1.

5. An elutriation chamber according to claim 3 in combination with claim 2.

6. A method for removing unwanted contaminants such as microparticles, microbial pathogens, plasma proteins, photodynamic and other virus-inactivating agents from thrombocytes, wherein an elutriation chamber according to any of claims 1 to 5 is used, with the proviso that the thrombocytes are not pelletized.

## Revendications

1. Chambre d'élutriation pour un système élutriateur pour le lavage et/ou l'isolement de cellules, en particulier de thrombocytes, dans laquelle ladite chambre d'élutriation comprend une conduite d'alimentation (1) qui est destinée à un milieu aqueux dans lequel les cellules à laver et/ou à isoler se trouvent en suspension, et présente une conduite d'évacuation (2) qui est destinée aux cellules lavées et/ou isolées, la chambre (5) étant de préférence symétrique en rotation par rapport à l'axe (a), **caractérisée en ce que** le rapport entre l'aire de la section de la lumière de la chambre (5) perpendiculairement à l'axe (a) à l'endroit le plus large (5a-5b) et l'aire de la section (1a) de la conduite d'alimentation (1) est compris entre 1 000 et 250 000.

2. Chambre d'élutriation pour un système élutriateur selon la revendication 1, **caractérisée en ce qu'**elle comprend une autre conduite d'alimentation (3) qui est destinée à un milieu gazeux ou liquide.

3. Chambre d'élutriation pour un système élutriateur pour le lavage et/ou l'isolement de cellules, en particulier de thrombocytes, dans laquelle ladite chambre d'élutriation comprend une conduite d'alimentation (1) circulaire en section transversale qui est destinée à un milieu aqueux dans lequel les cellules à laver et/ou à isoler se trouvent en suspension, et présente une conduite d'évacuation (2) qui est destinée aux cellules lavées et/ou isolées, **caractérisée en ce qu'**un rétrécissement (4), de préférence conique, est prévu à l'intérieur de la conduite d'alimentation (1) à l'entrée de la chambre, de telle sorte que le rapport entre l'aire de la section de la lumière de la chambre (5) perpendiculairement à l'axe (a) à l'endroit le plus large (5a-5b) et l'aire de la section du rétrécissement (4) est compris entre 1 000 et 250 000.

4. Chambre d'élutriation selon la revendication 3 prise en combinaison avec la revendication 1.

5. Chambre d'élutriation selon la revendication 3 prise en combinaison avec la revendication 2.

6. Procédé permettant d'éliminer des contaminants indésirables, tels que des microparticules, des pathogènes microbiens, des protéines plasmatiques, des agents photodynamiques ainsi que d'autres agents d'inactivation virale des thrombocytes, dans lequel une chambre d'élutriation est utilisée selon l'une quelconque des revendications 1 à 5, **caractérisé en ce que** les thrombocytes ne sont pas agglomérés sous la forme de boulettes.
